# EUROPEAN PATENT SPECIFICATION

(11) **EP 1 074 247 B1**
(45) Date of publication and mention of the grant of the patent: **27.09.2006**
(21) Application number: 00115548.0
(22) Date of filing: 19.07.2000
(51) Int. Cl.: A61K 8/27, A61K 8/46, A61Q 5/02, A61Q 15/00, A61Q 17/00, A61Q 19/00, C11D 1/14, C11D 1/29, C11D 3/00

(54) **Use of zinc alkylsulphates and/or alky(poly)ethoxysulphates as surfactants and preservatives in cleansing or cosmetic compositions**
Verwendung von Zink-alkylsulfaten und/oder -alkyl(poly)ethoxysulfaten als Tenside und Konservierungsmittel in Reiningungsmitteln oder kosmetischen Zusammensetzungen
Utilisation des alkylsulfates de zinc et/ou des alkylsulfates de zinc (poly) ethoxylés comme tensioactifs et conservateurs dans des compositions cosmétiques ou nettoyantes

(30) Priority: 20.07.1999 IT TO990639
(43) Date of publication of application: 07.02.2001
(73) Proprietor: Zschimmer & Schwarz Italiana S.p.A., 10038 Tricerro (Vercelli) (IT)
(72) Inventor: Ariotto, Angelo, 15030 Terruggia (Alessandria) (IT); Guala, Fabrizio, 13039 Trino (Vercelli) (IT)
(74) Representative: Comoglio, Elena

(56) References cited:
- WO-A-96/25913
- WO-A-97/26855
- WO-A-98/17195
- GB-A- 1 047 771
- US-A- 4 454 153
- US-A- 4 476 046
- US-A- 4 992 212
- US-A- 5 310 546
- US-A- 5 405 603
- US-A- 5 607 910
- DATABASE BIOSIS [Online] BIOSCIENCES INFORMATION SERVICE, PHILADELPHIA, PA, US; 1993 GLYNN T: "Benign footrot: An epidemiological investigation into the occurrence, effects on production, response to treatment and influence of environmental factors." Database accession no. PREV199396005851 XP002220189 -& AUSTRALIAN VETERINARY JOURNAL, vol. 70, no. 1, 1993, pages 7-12, XP009000317 ISSN: 0005-0423

## Description

The present invention relates to the use of zinc alkylsulphates and/or alkyl(poly)ethoxysulphates as surfactants and preservatives in cleansing or cosmetic compositions having a pH value less or equal to 6.

The use of alkylsulphates and alkylethoxysulphates as surfactants in toiletry compositions has been known for some time.

In particular, lauryl sulphate and lauryl ethoxysulphate, in the form of sodium or potassium salts or amines, are used widely in cleansing compositions such as shampoos (S. Giers and D. Boido, The Toilet Goods Assoc. 21:14-18 (1954)).

Since these compositions are easily attacked by microbial agents, the compositions of the prior art also contain preservatives which, however, have the disadvantage of having an irritant and/or sensitizing effect, particularly in relation to the skin, which effect is added to that of the alkyl sulphates and alkyl ethoxysulphates themselves.

The technical problem upon which the present invention is based is therefore to provide cleansing or cosmetic compositions which are free of skin irritant and/or sensitizing compounds but nevertheless have preservative properties.

It has surprisingly been found that zinc alkylsulphates and alkyl(poly)ethoxysulphates can advantageously be used as surfactants for solving the technical problem described above.

It has in fact been found from investigations performed by the Applicants that these surfactants have the same antiseptic properties as the inorganic Zn²⁺ cation and, if used in the formulation of cleansing or cosmetic compositions at sufficiently high concentrations, they can therefore preserve the compositions in which they are included. They also provide the compositions with anti-dandruff, sebonormalizing and deodorant properties.

Zinc alkylsulphates and alkyl(poly)ethoxysulphates are known compounds commonly used as catalysts in Diels-Alder reactions, as micellar solubilizers, and as fluidizers in cement preparations. They can be produced by salification of alkylsulphuric or alkyl(poly)ethoxysulphuric acids with zinc oxide (ZnO).

WO96/259B relates to the use of monophasic zinc hydroxycarbonate as antimicrobial agent in personal care products, particularly in such products which also contain a surfactant.

The subject of the present invention is therefore the use of a compound having the general formula

R- (OCH₂CH₂)ₘ-OSO₃⁻, ½Zn²⁺

in which R is saturated or unsaturated, branched or linear C₃-C₂₂ alkyl and m may vary from 0 to 20, as a surfactant and preservative in a cleansing or cosmetic composition having a pH value less or equal to 6.

Preferably, the composition is free of other preservatives or comprises at least one other preservative at a concentration which is ineffective and/or not sufficient to confer the desired characteristics of preservability in an analogous or similar composition which does not contain the said compound.

An "analogous or similar composition" is constituted by the same components in the same quantities as the compositions according to the invention described above, differing therefrom in that the surfactant and preservative having the general formula indicated above is replaced by an equal quantity of another anionic surfactant.

In general, a cleansing or cosmetic composition can be considered to have the desired characteristics of preservability when it responds positively to tests for assessing the efficacy of its preservative power. These tests are carried out in accordance with directions provided by the United States Pharmacopoea (USP) and/or by the Cosmetic, Toiletry and Fragrance Association (CTFA). These methods, and other similar methods, involve the inoculation of the sample with micro-organisms of various types and aerobic microbial counting of the micro-organisms at various times in order to assess their survival level.

The invention thus eliminates or in any case substantially reduces the concentration of the usual preservatives, the concentration of which, if they are present, will in any case be below the threshold of irritant and/or sensitizing activity.

The compounds defined by the general formula given above are known as zinc alkylsulphates (m = 0) or alkyl(poly)ethoxysulphates (m ≠ 0).

A use, wherein the composition also comprises at least one aqueous medium is preferred.

For the most preferred use, compositions are those in which R is lauryl.

The pH of the compositions according to the invention is preferably less than or equal to 6. Moreover, the concentration of the zinc alkylsulphates or alkyl(poly)ethoxysulphates to be used in the compositions according to the invention is preferably a concentration which is sufficient *per se* to confer the preservability characteristics, or is such as to confer the said characteristics in combination with another preservative which is present in concentrations which are not sufficient *per se* to confer the said characteristics.

This concentration is preferably between 5% and 25% by weight of active matter, relative to the total composition, more preferably between 5% and 15% by weight of active matter. Within this concentration range, the zinc alkylsulphates or alkyl(poly)ethoxysulphates can in fact themselves perform the preservative action.

The cleansing or cosmetic compositions according to the use of the invention may also comprise at least one further and different surfactant, selected from anionic surfactants such as, for example, alkylsulphate salts and alkylethersulphate salts, amido/amidoethersulphate salts, alkylsemisulphosuccinate and alkylsulphosuccinate salts, alkylethersemisulphosuccinate and alkylethersulphosuccinate salts, acylamidosemisulphosuccinate and acylamidosulphosuccinate salts, salts of acylated amino-acids (for example, acylsarcosinate salts), salts of acylated peptides and proteins, fatty-acid salts, salts of dodecylbenzenesulphonic acid, alkyl/alkylethersulphoacetate salts, salts of sulphonated and/or sulphated organic molecules (for example, α-olefin sulphonates) alkyl/alkylether carboxylate salts, alkylphosphonates, esters of phosphoric acid, acyl isethionate salts; amphoteric surfactants such as, for example, alkyl betaine, alkylamidopropyl betaine, amine and amide oxides, hydrolyzed proteins, amphocarboxyacetates and amphocarboxydiacetates, amphocarboxypropionates and amphocarboxydipropionates; non-ionic surfactants such as, for example, various amides, ethoxylated and non-ethoxylated fatty amines, ethoxylated nonylphenols, APGs (alkylpolyglucosides), AEGs (alkylethoxyglucosides), esters/ethers of fatty acids with glycerol and/or ethoxylated and non-ethoxylated sugars, various ethoxylated/propoxylated and non-ethoxylated/propoxylated esters, sorbitol, glycerol, various glycols, ethoxylated/propoxylated and non-ethoxylated/propoxylated fatty alcohols, silicone molecules; and cationic surfactants containing, for example, at least one quaternized N atom.

Amongst the preservatives which are commonly used in the cleansing or cosmetic compositions of the prior art but which are absent from the compositions according to the invention or, at most, are present in non-irritant or non-preservative concentrations as explained above are, for example, various aldehydes such as formaldehyde, chlorine, hypochlorites, chlorine-releasing compounds, chlorine dioxide, iodine and iodophors, phenol, cresol, chlorocresol, bisphenol, quaternary ammonium compounds, amphoteric compounds with preservative characteristics, chlorhexidine, peracetic and dehydroacetic acids, mercury compounds, alcohols, sorbic, benzoic, salicylic, boric, formic, propionic, undecylenic acids and their salts, bronopol, 5-bromo-5-nitrodioxane, hexamethylenetetramine, DMDM hydantoin, various hydantoins such as MDM hydantoin, chloracetamide, various ureas such as imidazolidinyl urea and diazolidinyl urea, inorganic sulphites, triclosan, parabens, various isothiazolinones, usnic acid and its salts, chlorophene, hexachlorophene, dichlorophene, bromochlorophene, triclocarban, cloflucarban, benzamidines which are classified as preservatives, amines which are classified as preservatives, dimethyl oxazolidine, ethyldicyclooxazolidine, dimethylhydroxymethyl pyrazole, polyaminopropyl biguanide, sodium hydroxymethyl glycinate, methyl dibromoglutaronitrile, glyceryl monolaurate and mixtures thereof.

With the use of suitable concentrations of the zinc alkylsulphates and/or alkyl(poly)ethoxysulphates described above, it is therefore possible to produce cleansing or cosmetic compositions which are gentle and safe with good performance and self-preservative properties but which are free of other preservatives, or in which these other preservatives are present at most in non-irritant and/or non-sensitizing or non-preservative concentrations.

For this purpose, the use of the compound, preferably zinc laurylethoxysulphate, in combination with a salt of an acylated amino-acid or peptide derivative is most preferred.

The salt is preferably a salt of an alkali metal such as sodium, potassium or lithium, or of an alkaline-earth metal such as magnesium, of ammonia, or of an amine base such as TEA (triethanolamine), MEA (monoethanolamine), DEA (diethanolamine), arginine, lysine or AMP (aminomethylpropanol).

The acyl group preferably has 8 carbon atoms and most preferably is capryloyl.

The amino-acid is preferably selected from the group which consists of glutamic acid, aspartic acid, glycine, lysine, serine, proline, arginine, PCA (pyrrolidone carboxylic acid), tryptophan, glutamine, asparagine, threonine, valine, cysteine, tyrosine, phenylalanine, methionine, leucine, isoleucine, histidine, and hydroxyproline; the peptide is preferably selected from wheat and collagen peptides.

The compositions thus formulated in fact have good self-preservative characteristics and a low irritant effect and at the same time exhibit a good deodorant effect.

The deodorant efficacy of compositions produced with the use of these compounds was assessed by a sniff test described in detail in Example 15 below.

Some products in which the compositions of the invention may be used are: shampoos, foam baths, shower foam, dishwashing detergents, liquid soaps, solid soaps, lotions, emulsions of various kinds, balsams, products having both cleansing and skin-conditioning and/or hair-conditioning effects, oils, emollient and cleansing milks, face and/or body and/or hair creams, personal hygiene cleansers, brilliantines, permanent hair-waving solutions, hair-dyes, toothpastes, medicated cosmetics, and pharmaceutical products.

The following examples describe the formulations of some products, wherein percentages are given by weight. They are provided exclusively by way of illustration.

### EXAMPLES

### Example 1: Foam bath for children (preservative-free)

zinc laurylethoxysulphate ethoxylate 3 moles OE (25% active matter): 40%
acylated hydrolyzed wheat protein, sodium salt (28% active matter) : 2%
citric acid: as much as necessary
NaCl: as much as necessary
colouring and perfume: as much as necessary
water: to 100%

### Example 2: gentle foam bath (preservative-free)

zinc laurylethoxysulphate ethoxylate 3 moles OE (25% active matter): 30%
lauramidopropyl betaine (30% active matter): 4%
sodium lauroyl glutamate (30% active matter): 2%
PEG-7 glyceryl cocoate: 0.5%
citric acid: as much as necessary
NaCl, colouring, perfume and water: to 100%

### Example 3: shower bath (preservative-free)

zinc laurylethoxysulphate ethoxylate 3 moles OE (25% active matter): 30%
sodium cocoamphodiacetate (26% active matter): 15% triethanolamine lauryl sulphate (39% active matter): 6% citric acid: as much as necessary
NaCl, colouring, perfume and water: to 100%

### Example 4: shower gel with deodorant effect

zinc laurylethoxysulphate ethoxylate 3 moles OE (25% active matter): 40%
lauramidopropyl betaine (30% active matter): 10% sodium semisulphosuccinate (di-sodium laurylethoxysulphosuccinate (30% active matter): 6%
citric acid: as much as necessary
NaCl, colouring, perfume and water: to 100%

### Example 5: shampoo for greasy hair

zinc laurylethoxysulphate ethoxylate 3 moles OE (25% active matter) : 25%
sodium cocoamphodiacetate (26% active matter): 10% triethanolamine lauryl sulphate (39% active matter): 5%
coco-amide: 1%
citric acid: as much as necessary
NaCl, colouring, perfume and water: to 100%

### Example 6: shampoo for greasy hair

zinc laurylethoxysulphate ethoxylate 3 moles OE (25% active matter): 30%
lauramidopropyl betaine (30% active matter): 4%
sodium lauryl sulphate (28% active matter): 7% acylated hydrolyzed wheat protein, sodium salt (28% active matter: 4%
citric acid: as much as necessary
NaCl, colouring, perfume and water: to 100%

### Example 7: anti-dandruff shampoo

zinc laurylethoxysulphate ethoxylate 3 moles OE (25% active matter): 40%
coco-amide: 2%
monoethanolamine lauryl sulphate (27% active matter): 5% citric acid: as much as necessary
NaCl, colouring, perfume and water: to 100%

### Example 8: anti-dandruff shampoo

zinc laurylethoxysulphate ethoxylate 3 moles OE (25% active matter): 45%
cocamidopropyl betaine oxide (30% active matter): 4%
coco-amide: 2%
citric acid: as much as necessary
NaCl, colouring, perfume and water: to 100%

### Example 9: sebonormalizing face-wash gel

zinc laurylethoxysulphate ethoxylate 3 moles OE (25% active matter): 25%
sodium lauroyl glutamate (30% active matter): 10%
citric acid: as much as necessary
colouring, perfume, excipients and water: to 100%

### Example 10: sebonormalizing face-wash gel

composition according to Example 9 in which sodium lauroyl glutamate is replaced by sodium octanoyl glutamate.

### Example 11: sebonormalizing face-wash gel

zinc laurylethoxysulphate ethoxylate 3 moles OE (25% active matter): 30%
sodium lauroyl sarcosinate (29% active matter): 5%
citric acid: as much as necessary
colouring, perfume, excipients and water: to 100%

### Example 12: sebonormalizing face-wash gel

composition according to Example 11, in which sodium lauroyl sarcosinate is replaced by sodium octanoyl sarcosinate.

### Example 13: dishwashing detergent

zinc laurylethoxysulphate ethoxylate 3 moles OE (25% active matter): 25%
sodium dodecylbenzenesulphonate (30% active matter): 25% coco-amide: 2%
citric acid: as much as necessary
NaCl, colouring, perfume and water: to 100%

### Example 14: toothpaste

zinc lauryl sulphate (25% active matter): 1.5%
sodium monofluorophosphate: 0.78%
silica zerogel: 14%
carboxymethyl cellulose: 0.3%
hydrated silica: 8%
polyethylene glycol 1450: 5%
sorbitol 70%: 46.7%
glycerol: 21%
aroma: 2%
saccharine: 0.2%
colouring: 0.52%

### Example 15: assessment of deodorant effect

The deodorant effect of a composition comprising zinc laurylethoxysulphate was assessed by a sniff test carried out on a group of 12 volunteers.

For this purpose, the following aqueous solutions were prepared:
- "Sample A": zinc laurylethoxysulphate, 12% active matter,
- "Sample B": sodium laurylethoxysulphate, 12% active matter and 0.5% of triclosan (trichlorohydroxydiphenyl ether),
- "Sample C": zinc laurylethoxysulphate, 11% active matter and sodium capryloyl glutamate, 1% active matter.

Triclosan is a deodorant and preservative which is known and commonly used in the formulation of cleansing or cosmetic compositions. Sample B is therefore a cleansing and deodorant composition according to the prior art which was used as a control in the sniff test described below.

### Sniff test

The volunteers were subjected to a week of preconditioning (Stage 1) during which they were required not to use deodorants or antibacterial soaps and to wash with the use exclusively of the cleansing compositions indicated above.

Upon completion of the preconditioning, a sniff test, on the basis of which the participants were selected for the test, was performed, 6 hours after the last wash.

Subjects who had an odour which was too strong (intensity 10), too faint (intensity 1), or imperceptible (intensity 0) were discarded.

Each subject then used the product under test for two weeks. At the end of the period of use a sniff test was performed 6 hours and 24 hours after the last application (Stage 2 and Stage 3, respectively).

The odour was assessed by a panel of 3 expert assessors with the use of a linear scale of intensity from 0 to 10, in which:
- the value 0 indicated no odour,
- the value 10 indicated a very strong odour.

The results are expressed as a reduction in odour relative to the base value.

Before assessment, the subject was left to acclimatize for 15 minutes in an air-conditioned room (T 24°C, relative humidity 50%).

Armpit odour was assessed by 3 odour assessors on the basis of the following procedure:
- the subject raised his arm (first the right arm and then the left arm);
- the assessors sniffed, one at a time and independently of one another. For correct odour assessment, the assessor brought his nose to a distance of 5 cm from the centre of the subject's armpit, recording a score corresponding to the intensity perceived, on a suitable card.

During the test, both the assessors and the laboratory personnel present in the room had to avoid smoking or eating for at least 30 minutes before the start of the assessment. The odour assessors were also required not to apply perfumes, colognes, after-shave or any products which might influence the outcome of the test in any way.

For each sample and each stage of the investigation, the mean value of the opinions expressed by the 3 odour assessors was calculated for each individual subject.

The arithmetic mean and the standard deviation were then calculated for the three sets of data.

The data were then expressed as follows:
% decrease 6 hours after the last application, defined as:
   [(T₀ - T₆) /T₀] x 100; and
% decrease 24 hours after the last application, defined as:
   [(T₀ - T₂₄) /T₀] x 100,
where:
T₀ = mean odour assessment after the preconditioning period (Stage 1);
T₆ = mean odour assessment 6 hours after the last application of the product (Stage 2);
T₂₄ = mean odour assessment 24 hours after the last application of the product (Stage 3).

In order to check the deodorant efficacy of each product, the initial values (T₀) and the final values (T₆ and T₂₄) were compared statistically by t-tests for matched data.

The difference between the two sets of data analyzed was considered significant for a probability factor p ≤ 0.05.

Moreover, analysis of variance and the Turkey test were performed in order to produce evidence of statistically significant differences between the three samples tested. The difference between the sets of data analyzed was considered significant for a probability value p ≤ 0.05.

The results of the sniff test obtained with the three cleansing compositions subjected to the test are shown in Tables 1A, 1B and 1C below.

**Table 1A**

| Sample A - decrease in armpit odour and statistical analysis after 6 and 24 hours | | |
|---|---|---|
| | % decrease after 6 hours | % decrease after 24 hours |
| mean | 55.7% | 37.4% |
| standard deviation | 39.3 | 43.9 |
| t-test | p = 0.002 | p = 0.005 |

**Table 1B**

| Sample B - decrease in armpit odour and statistical analysis after 6 and 24 hours | | |
|---|---|---|
| | % decrease after 6 hours | % decrease after 24 hours |
| mean | 47.4% | 35.3% |
| standard deviation | 53.9 | 42.5 |
| t-test | p = 0.005 | p = 0.005 |

**Table 1C**

| Sample C - decrease in armpit odour and statistical analysis after 6 and 24 hours | | |
|---|---|---|
| | % decrease after 6 hours | % decrease after 24 hours |
| mean | 79.8% | 70.0% |
| standard deviation | 27.2 | 35.8 |
| t-test | p < 0.001 | p < 0.001 |

### Conclusions

In order to assess the deodorant efficacy, each of Sample A, Sample B, and Sample C was used by 12 volunteers.

At the end of the set period of use, sniff assessments of armpit odour were performed 6 and 24 hours after the last application of the product.

On the basis of the results obtained, it can be stated that:
- Sample A brought about a significant reduction in the basic mean odour level both 6 hours (55.7%) and 24 hours (37.4%) after the last application;
- Sample B brought about a significant reduction in armpit odour both 6 hours (47.4%) and 24 hours (35.3%) after the last application;
- Sample C brought about a highly significant reduction in armpit odour both 6 hours (79.8%) and 24 hours (70.0%) after the last application.

The statistical comparison performed by analysis of variance did not show any significant difference between the three samples tested. The deodorant efficacy of the three products was therefore of comparable magnitude.

## Claims

1. Use of a compound having the general formula
R- (OCH₂CH₂)ₘ-OSO₃⁻·½Zn²⁺
in which R is saturated or unsaturated, branched or linear C₃-C₂₂ alkyl and m may vary from 0 to 20, as a surfactant and preservative in a cleansing or cosmetic composition having a pH value less or equal to 6.

2. Use according to Claim 1, wherein the composition is free of other preservatives or comprises at least another preservative at a concentration which is ineffective and/or not sufficient to confer the desired characteristics of preservability in an analogous or similar composition which does not contain the said compound.

3. Use according to Claim 2, wherein the other preservative is selected from the group consisting of formaldehyde, chlorine, hypochlorites, chlorine-releasing compounds, chlorine dioxide, iodine and iodophors, phenol, cresol, chlorocresol, bisphenol, quaternary ammonium compounds, amphoteric compounds with preservative characteristics, chlorhexidine, peracetic and dehydroacetic acid, mercury compounds, alcohols, sorbic, benzoic, salicylic, boric, formic, propionic, undecylenic acids and their salts, bronopol, 5-bromo-5-nitrodioxane, hexamethylenetetramine, DMDM hydantoin, various hydantoins such as MDM hydantoin, chloracetamide, various ureas such as imidazolidinyl urea and diazolidinyl urea, inorganic sulphites, triclosan, parabens, various isothiazolinones, usnic acid and its salts, chlorophene, hexachlorophene, dichlorophene, bromochlorophene, triclocarban, cloflucarban, benzamidines which are classified as preservatives, amines which are classified as preservatives, dimethyl oxazolidine, ethyldicyclo-oxazolidine, dimethylhydroxymethyl pyrazole, polyaminopropyl biguanide, sodium hydroxymethyl glycinate, methyl dibromoglutaronitrile, glyceryl monolaurate and mixtures thereof.

4. use according to any of Claims 1 to 3, wherein the composition also comprises an aqueous medium.

5. use according to any of Claims 1 to 4, wherein R is lauryl.

6. use according to any of Claims 1 to 5, wherein the compound is used at a concentration of between 5% and 25% by weight of active matter relative to the total composition.

7. Use according to Claim 6, wherein the concentration is between 5% and 15% by weight of active matter relative to the total composition.

8. Use according to any one of Claims 1 to 7, wherein the composition also comprises at least one further and different surfactant selected from anionic, cationic, non-ionic and amphoteric surfactants and mixtures thereof.

9. Use according to any of Claims 1 to 8, wherein the compound also has a deodorant effect.

10. Use according to any of Claim 1 to 9, wherein the compound is zinc laurylethoxysulphate and wherein the composition also comprises a further and different surfactant which is a salt of an acylated amino-acid or peptide derivative having an acyl group with 8 carbon atoms.

## Patentansprüche

1. Verwendung einer Verbindung mit der allgemeinen Formel
R-(OCH₂CH₂)ₘ-OSO₃⁻•¹/₂Zn²⁺,
wobei R ein gesättigtes oder ungesättigtes, verzweigtes oder lineares C₃-C₂₂-Alkyl ist und m von 0 bis 20 variieren kann,
als Tensid und Konservierungsmittel in einer Reinigungs- oder Kosmetikzusammensetzung mit einem pH-Wert, der kleiner oder gleich 6 ist.

2. Verwendung gemäß Anspruch 1, wobei die Zusammensetzung frei von anderen Konservierungsmitteln ist oder zumindest ein anderes Konservierungsmittel in einer Konzentration umfasst, die unwirksam ist und/oder nicht ausreicht, um die gewünschten Eigenschaften der Konservierbarkeit in einer analogen oder ähnlichen Zusammensetzung, welche die besagte Verbindung nicht enthält, zu verleihen.

3. Verwendung gemäß Anspruch 2, wobei das andere Konservierungsmittel ausgewählt ist aus der Gruppe, bestehend aus Formaldehyd, Chlor, Hypochloriten, Chlor freisetzenden Verbindungen, Chlordioxid, Iod und Iodophoren, Phenol, Kresol, Chlorkresol, Bisphenol, quartären Ammoniumverbindungen, amphoteren Verbindungen mit konservierenden Eigenschaften, Chlorhexidin, Peressig- und Dehydroessigsäure, Quecksilberverbindungen, Alkoholen, Sorbin-, Benzoe-, Salicyl-, Bor-, Ameisen-, Propion-, Undecylensäure und deren Salzen, Bronopol, 5-Brom-5-nitrodioxan, Hexamethylentetramin, DMDM-Hydantoin, verschiedenen Hydantoinen wie z.B. MDM-Hydantoin, Chloracetamid, verschiedenen Harnstoffen wie z.B. Imidazolidinylharnstoff und Diazolidinylharnstoff, anorganischen Sulfiten, Triclosan, Parabenen, verschiedenen Isothiazolinonen, Usninsäure und deren Salzen, Chlorophen, Hexachlorophen, Dichlorophen, Bromchlorophen, Triclocarban, Cloflucarban, Benzamidinen, die als Konservierungsmittel eingestuft sind, Aminen, die als Konservierungsmittel eingestuft sind, Dimethyloxazolidin, Ethyldicyclooxazolidin, Dimethylhydroxymethylpyrazol, Polyaminopropylbiguanid, Natriumhydroxymethylglycinat, Methyldibromglutaronitril, Glycerylmonolaurat und Mischungen davon.

4. Verwendung gemäß einem der Ansprüche 1 bis 3, wobei die Zusammensetzung auch ein wässriges Medium umfasst.

5. Verwendung gemäß einem der Ansprüche 1 bis 4, wobei R Lauryl ist.

6. Verwendung gemäß einem der Ansprüche 1 bis 5, wobei die Verbindung in einer Konzentration zwischen 5 und 25 Gew.% aktiver Stoff, bezogen auf die gesamte Zusammensetzung, verwendet wird.

7. Verwendung gemäß Anspruch 6, wobei die Konzentration zwischen 5 und 15 Gew.% aktiver Stoff, bezogen auf die gesamte Zusammensetzung, beträgt.

8. Verwendung gemäß einem der Ansprüche 1 bis 7, wobei die Zusammensetzung auch zumindest ein weiteres und unterschiedliches Tensid, ausgewählt aus anionischen, kationischen, nichtionischen und amphoteren Tensiden und Mischungen davon, umfasst.

9. Verwendung gemäß einem der Ansprüche 1 bis 8, wobei die Verbindung auch eine geruchsbeseitigende Wirkung aufweist.

10. Verwendung gemäß einem der Ansprüche 1 bis 9, wobei die Verbindung Zinklaurylethoxysulfat ist und wobei die Zusammensetzung auch ein weiteres und unterschiedliches Tensid umfasst, das ein Salz einer acylierten Aminosäure oder ein Peptidderivat mit einer Acylgruppe mit 8 Kohlenstoffatomen ist.

## Revendications

1. Utilisation d'un composé ayant la formule générale :
R-(OCH₂CH₂)ₘ-OSO₃⁻.½Zn²⁺
dans laquelle R est un groupe alkyle en C₃-C₂₂ saturé ou insaturé, ramifié ou linéaire, et m peut varier de 0 à 20, en tant que tensioactif et conservateur dans une composition nettoyante ou cosmétique ayant une valeur de pH inférieure ou égale à 6.

2. Utilisation selon la revendication 1, dans laquelle la composition est exempte d'autres conservateurs, ou comprend au moins un autre conservateur à une concentration qui est inefficace et/ou insuffisante pour conférer les caractéristiques requises d'aptitude à la préservation à une composition analogue ou similaire qui ne contient pas ledit composé.

3. Utilisation selon la revendication 2, dans laquelle l'autre conservateur est choisi parmi le groupe consistant en le formaldéhyde, le chlore, les hypochlorites, les composés libérant du chlore, le dioxyde de chlore, l'iode et les iodophores, le phénol, le crésol, le chlorocrésol, le bisphénol, les composés de type ammonium quaternaire, les composés amphotères ayant des caractéristiques préservatrices, la chlorhexidine, les acides peracétiques et déshydroacétiques, les composés du mercure, les alcools, les acides sorbique, benzoïque, salicylique, borique, formique, propionique et undécylénique et leurs sels, le bronopol, le 5-bromo-5-nitrodioxane, l'hexaméthylènetétramine, la DMDM hydantoïne, diverses hydantoïnes telles que la MDM hydantoïne, le chloracétamide, diverses urées telles que l'imidazolidinyl urée et la diazolidinyl urée, les sulfites inorganiques, le trichlosan, les parahydroxybenzoates, diverses isothiazolinones, l'acide usnique et ses sels, le chlorophène, l'hexachlorophène, le dichlorophène, le bromochlorophène, le trichlocarban, le chloflucarban, les benzamidines classifiées en tant que conservateurs, les amines classifiées en tant que conservateurs, la diméthyl oxazolidine, l'éthyldicyclo-oxazolidine, le diméthylhydroxyméthyl pyrazole, le polyaminopropyl biguanide, l'hydroxyméthyl glycinate de sodium, le méthyl dibromoglutaronitrile, le monolaurate de glycéryle et leurs mélanges.

4. Utilisation selon l'une quelconque des revendications 1 à 3, dans laquelle la composition comprend également un milieu aqueux.

5. Utilisation selon l'une quelconque des revendications 1 à 4, dans laquelle R représente un groupe lauryle.

6. Utilisation selon l'une quelconque des revendications 1 à 5, dans laquelle le composé est employé selon une concentration de 5 % à 25 % en poids de matière active par rapport à la composition totale.

7. Utilisation selon la revendication 6, dans laquelle la concentration est de 5 % à 15 % en poids de matière active par rapport à la composition totale.

8. Utilisation selon l'une quelconque des revendications 1 à 7, dans laquelle la composition comprend également au moins un autre tensioactif différent choisi parmi les tensioactifs anioniques, cationiques, non ioniques et amphotères et leurs mélanges.

9. Utilisation selon l'une quelconque des revendications 1 à 8, dans laquelle le composé a également effet déodorant.

10. Utilisation selon l'une quelconque des revendications 1 à 9, dans laquelle le composé est le lauryléthoxysulfate de zinc, et dans laquelle la composition comprend également un autre tensioactif différent qui est un sel d'un dérivé acylé d'aminoacide ou de peptide comportant un groupe acyle à 8 atomes de carbone.
